# EUROPÄISCHE PATENTSCHRIFT

(11) **EP 2 349 194 B1**
(45) Veröffentlichungstag und Bekanntmachung des Hinweises auf die Patenterteilung: **21.03.2018**
(21) Anmeldenummer: 09748242.6
(22) Anmeldetag: 23.10.2009
(51) Int. Cl.: A61K 8/891, A61K 8/41, A61K 8/34, A61K 8/898, A61Q 5/12

(54) **SYNERGISTISCHE MISCHUNGEN VON KATIONISCHEM AMINOSILIKON TERPOLYMER MIT EINEM WEITEREN SILIKON**
SYNERGISTIC MIXTURES OF A CATIONIC AMINO SILICONE TERPOLYMER AND ANOTHER SILICONE
MÉLANGES SYNERGIQUES COMPRENANT UN TERPOLYMÈRE D'AMINOSILICONE CATIONIQUE ET UNE AUTRE SILICONE

(30) Priorität: 23.10.2008 DE 102008052859
(43) Veröffentlichungstag der Anmeldung: 03.08.2011
(73) Patentinhaber: Beiersdorf AG, 20245 Hamburg (DE)
(72) Erfinder: SALADIN, Sandra, 20144 Hamburg (DE); MAHADESHWAR, Anand, 07442 New Jersey (US); KRAUSS, Nicole Maria, 20149 Hamburg (DE)
(86) Internationale Anmeldenummer: PCT/EP2009/007598
(87) Internationale Veröffentlichungsnummer: WO 2010/046119

(56) Entgegenhaltungen:
- EP-A1- 1 920 758
- EP-A1- 2 022 471
- EP-A1- 2 022 478
- EP-A1- 2 161 015
- WO-A1-02/10257
- WO-A1-2004/105710
- WO-A2-2004/069137
- DOW CORNING: "DOW CORNING 5-7113 SILICONE QUAT MICROEMULSION" PRODUCT INFORMATION PERSONAL CARE DOW CORNING,, 13. November 2006 (2006-11-13), Seiten 1-4, XP007910346
- DOW CORNING: "DOW CORNING TM 5-7070 Si Amino Elastomer Emulsion" PRODUCT INFORMATION PERSONAL CARE DOW CORNING,, 15. Juli 2008 (2008-07-15), Seiten 1-6, XP007910347

## Beschreibung

Haarspülungen werden zur Pflege nach der Reinigung der Haare - einer Form keratinischer Fasern - angewendet. Sie sind normalerweise auf Basis von kationischen Tensiden wie beispielsweise Cetyl Trimethyl Ammonium Chlorid und Fettalkoholen wie Cetyl und/oder Stearylalkohol. Allein reicht diese Kombination jedoch nicht für eine geeignete konditionierende Pflege an Haaren wie unter anderem Geschmeidigkeit, Kämmbarkeit und Glanz aus. Aus diesem Grund werden häufiger Silikone wie beispielsweise langkettige Polydimethylsiloxane eingesetzt. Noch substantiver sind Silikone, die Aminogruppen enthalten. In einer Spülungsformulierung bei pH-Werten von 4 - 5 sind diese Gruppen protoniert, so dass die kationische Natur des Silikons zu einer erhöhten Konditionierleistung führt. Jedoch ist die Kernseite diese Eigenschaft eine Neigung zur statischen Aufladung der Haare. Somit besteht die Notwendigkeit konditionierende Formulierungen, die eine überragende konditionierende Leistung hinsichtlich Geschmeidigkeit und Kämmbarkeit bringen können ohne den Nachteil der statischen Aufladung der Haare zu entwickeln. WO-2004069137 A2 beschreibt die Verwendung von Polyammonium Polysiloxancopolymeren. Eine Spülungsformel mit einem kationischen Aminosilikon Terpolymer führt zu einer überlegenen Pflege hinsichtlich des Gleitvermögens im nassen Haar, Nasskämmbarkeit und Haarstruktur, jedoch verliert in den Eigenschaften des trockenen Haares, besonders durch die extreme statistische Aufladung.

Die Schrift EP-1309649B1 offenbart bestimmte Polysiloxan-Verbindungen mit Polyalkylenoxid-Struktureinheiten, mindestens einen zweiwertigen oder dreiwertigen organischen Rest, der mindestens eine Ammoniumgruppe enthält, mindestens eine Polysiloxan-Struktureinheit, mindestens einen organischen oder anorganischen Säurerest zur Neutralisation der aus der(n) Ammonium-gruppe(n) resultierenden Ladungen.

Die Schrift WO-2004069137 A2 offenbart die Verwendung mindestens eines bestimmten linearen oder verzweigten Polyamino-und/oder Polyammonium-Polysiloxancopolymer in der Herstellung und/oder Behandlung von gefärbtem Haar.

Die Schrift EP-1920758 A1 offenbart Reinigungszubereitungen für keratinische Fasern mit bestimmten Tensiden, C12-22 Monoglyceriden und Silikonpolymeren mit quternärer Ammoniumgruppe und Polyethergruppe.

All dies konnte nicht den Weg zur vorliegenden Erfindung weisen. Es hat sich überraschend herausgestellt, dass eine Zubereitung zur Behandlung keratinischer Fasern enthaltend a) ein kationisches Aminosilikon Terpolymer mit quaternären Ammonium-Gruppen und Polyethergruppen, b) ein Dimethikon und/oder ein zweites Aminosilikon, c) ein kationisches Tensid, d) eine oder mehrere Fettverbindungen mit Schmelzpunkt > 40°C und einen wässrigen Träger, wobei als kationisches Aminosilikon Terpolymer mit quaternären Ammonium-Gruppen und Polyethergruppen Silikon Quaternium-18 gewählt wird und als Dimethikon ein hochmolekulares Dimethikon mit einer Viskosität von 60.000 bis 500.000 cSt gemessen mit einem Brookfield Viscosimeter RVF, RVT oder RVDV-II + gewählt wird, den Nachteilen des Standes der Technik abhilft.

Es hat sich gezeigt, dass die Kombination von einem kationischen Aminosilikon Terpolymer mit einem weiteren Silikon zu einer überragenden Haarkonditionierung im nassen und trockenen Haar führt ohne den Nachteil von statischer Aufladung. Dabei weist das hochmolekulare Dimethikon eine Viskosität von 60.000 cSt bis 100.000 cSt, bevorzugt von 100.000 cSt bis 500.000 cSt gemessen mit einem Brookfield Viscosimeter RVF, RVT oder RVDV-II + auf. Auch ist es bevorzugt, wenn das zweite Aminosilikon eine Viskosität von 1000cst bis 6000cst, vorzugsweise von 1000cst bis 5000cst gemessen mit Brookfield Viscosimeter RVF, RVT oder RVDV-II + aufweist. Die folgenden Handelsnamen beziehen sich auf das jeweils zum Zeitpunkt der Erstanmeldung auf dem Markt erhältliche Produkt. Dabei ist als kationisches Aminosilikon Terpolymer mit quaternären Ammonium-Gruppen und Polyethergruppen Silicone Quaternium-18 auszuwählen, welches unter den von Momentive angebotenen Handelsnamen Silsoft Q und Silsoft Care bekannt ist. Dabei ist es bevorzugt, wenn als Dimethikon Dow Corning 200 Fluid 60,000cst, Dow Corning 5-7139 Emulsion, sowie auch Gum in Fluid Emulsionen nach Dow Corning 2-1352 und Dow Corning 2-1491 verwendet werden. Dabei ist es bevorzugt, wenn als zweites Aminosilikon Fluide nach Dow Corning 2-8566, Dow Corning AP-6087, Dow Corning AP-8087 verwendet werden. Dabei ist es bevorzugt, wenn als kationisches Tensid bevorzugt Stearamidopropyl Dimethylamine verwendet wird. Dabei ist es bevorzugt, wenn das kationische Aminosilikon Terpolymer mit quaternären Ammonium-Gruppen und Polyethergruppen in Konzentrationen von 0.25 bis 1.5 Gew.-%, bevorzugt von 0.5 bis 1.0 Gew.-%, eingesetzt wird, besonders bevorzugt ist genau dabei, wenn das Dimethikon und/oder ein zweites Aminosilikon in gleichen oder höheren Konzentrationen, bevorzugt 1.5:1, 2:1, als das kationische Aminosilikon Terpolymer mit quaternären Ammonium-Gruppen und Polyethergruppen, jeweils bezogen auf den Aktivgehalt, eingesetzt wird. Alle Konzentrationsangaben dieser Schrift sind auf den mit der Aktivgehalt bezogen. Die Erfindung umfasst auch die Verwendung von Dimethikon und/oder einem zweiten Aminosilikon nach einem der vorangehenden Ansprüche zur Verringerung der statischen Aufladung von mit kationischem Aminosilikon Terpolymer mit quaternären Ammonium-Gruppen und Polyethergruppen nach einem der vorangehenden Ansprüche, kationischem Tensid nach einem der vorangehenden Ansprüche und wenigstens einer Fettverbindung mit Schmelzpunkt > 40°C nach einem der vorangehenden Ansprüche in einem wässrigen Träger behandelten keratinischen Fasern, insbesondere menschliche Haare.

Ein anderer Gegenstand betrifft die Verringerung der Trocknungszeit von Haarbehandlungszubereitungen und die Erhöhung des Farbschutzes des gefärbten Haares, wie durch Ausbleichen oder Auslaugen der Haarfarbe. Die Kombination des Silicone Quaternium-16 von Dow Corning (Handelsname: Dow Corning 5-7113 silicone quat Microemulsion) mit Stearamidopropyl Dimethylamine von Evonik Goldschmidt/Evonik Degussa (Handelsname: Tego Amid S 18) und Dimethicone von Dow Corning (Handelsname: Dow Corning 5-7139_RMT) kann man eine signifikant bessere Kämmbarkeit im trockenen Haar erzielen, sowie eine signifikant schnellere Trocknung des Haares. Ebenso wird der Auswaschschutz von Haarfarbe deutlich erhöht.

Es ist bekannt, dass kationische Amine, insbesondere tertiäre Amine in Kombination mit Fettalkoholen wie Cetyl und Stearyl Alkohol zu einer reichhaltigen, cremigen Spülungsgrundlage führen. Behandlung einer Haarsträhne (2 Std. 2g gebleicht, 23cm lang; Firma Kerling) mit einer Tertiär-Amin basierten Spülung (Formel 1), die 1.5% Silsoft Q (Silicone Quaternium-18) enthält, führte zu einer starken statischen Aufladung nach 1x und besonders nach 3x Kämmen mit der feinen Seite eines Kamms (Typ 623.7 Hercules Sägemann ® 394.7), wie in Bild 1 abgebildet. Strähnen, die mit 1.5% Dimethikon sowie auch mit einer Mischung von 1.0% Dimethikon und 0.5% aktiv Silsoft Q aus Spülungsformeln behandelt wurden, zeigten hingegegen wenig Aufladung und die Haarsträhnen blieben parallel.

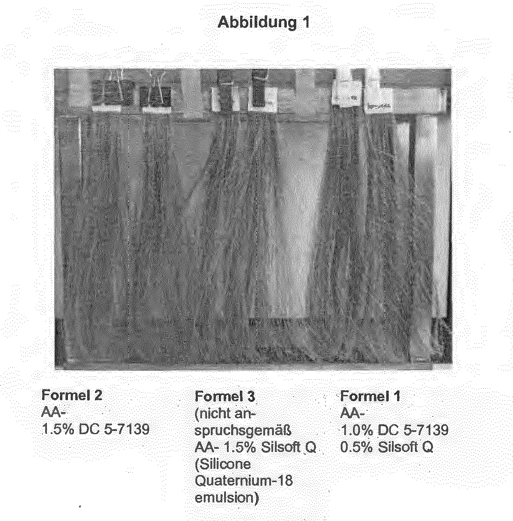

### Bild 1 (siehe Abbildung 1)

Vergleich Silsoft Q (2 Strähnen mitte, Formel 3) mit einem hochmolekülaren Dimethikon (DC 5-7139) (2 Strähnen links, Formel 2) und einer Mischung von hochmolekülarem Dimethikon und Silsoft Q (2 Strähnen rechts, Formel 1) nach Spülungsbehandlungen. - 3x Kämmen Formeln **1** und **2** (Tabelle 1) wurden im Halbseitentest im Haarstudio an trockenstrapazierten Haaren (N=10 Probanden) getestet und die Ergebnisse sind in Tabelle 2 abgebildet.

| | inci | **1** | **2** | **3** |
|---|---|---|---|---|
| 1 | Methylparaben | 0,25 | 0,25 | 0,25 |
| 2 | Propylparaben | 0,10 | 0,10 | 0,10 |
| 3 | Aqua | 83,70 | 85,43 | 80,24 |
| 4 | Cetyl Alcohol | 2,50 | 2,50 | 2,50 |
| 5 | Lactic Acid | 0,80 | 0,80 | 0,80 |
| 6 | Sodium Chloride | 0,01 | 0,01 | 0,01 |
| 7 | Stearyl Alcohol | 4,80 | 4,80 | 4,80 |
| 8 | Oryzanol | 0,05 | 0,05 | 0,05 |
| 9 | Coco Betaine | 0,85 | 0,85 | 0,85 |
| 10 | Stearamidopropyl Dimethylamine | 2,20 | 2,20 | 2,20 |
| 11 | Water (and) Silicone Quaternium-18 | 2,50 | | 7,50 |
| | (and) Trideceth-6 (and) Trideceth-12 | | | |
| 12 | Parfum | 0,70 | 0,70 | 0,70 |
| 13 | Dimethicone + Cocamidopropyl Betaine + C12-15 Pareth-3 + Guar Hydroxypropyltrimonium Chloride | 1,54 | 2,31 | |

| **Tabelle 1** | **Handelsnamen der Rohstoffe:** |
|---|---|
| 1 | Nipagin M, Clariant, 99.5% |
| 2 | Nipasol M, Clariant, 99.5% |
| 4 | Nacol 16-95, Sasol, 95% |
| 5 | 100366 Milchsäure 90, reinst DAB, Merck, 90% |
| 7 | Lanette 18, Cognis, 100% |
| 8 | Gamma -Oryzanol, Biesterfeld, 98% |
| 9 | Dehyton AB 30, Cognis, 31% |
| 10 | Tego Amid S18, Evonik Goldschmidt, 100% |
| 11 | Silsoft Q, Momentive Performance Materials, 20% |
| 13 | DC 5-7139, 65% Dimethicone, Dow Corning |

| **Tabelle 2: Haarstudio Daten; Halbseiten-Test an trocken/ strapazierten Haaren, N=10 Probanden** | | |
|---|---|---|
| | **2** | **1** |
| Gehalt verteilen | 6,20 | 7,00 |
| Gleitvermögen verteilen | 6,30 | 7,30 |
| Gehalt sofort Ausspülen | 4,90 | 5,50 |
| Gleitvermögen sofort Ausspülen | 6,70 | 7,30 |
| Entwirrbarkeit nass | 6,20 | 6,90 |
| Kämmbarkeit nass | 6,80 | 7,70 |
| Haarstruktur nass Längen | 6,80 | 7,10 |
| Haarstruktur nass Spitzen | 5,60 | 6,10 |
| Gleitvermögen nass | 6,30 | 7,00 |
| Entwirrbarkeit trocken | 7,00 | 7,20 |
| Kämmbarkeit trocken | 7,50 | 7,90 |
| Gleitfähigkeit trocken | 7,00 | 7,30 |
| Struktur trocken Längen | 7,70 | 7,60 |
| weich, geschmeidig trocken | 1,00 | 4,00 |

Die Silikonmischung von 1.0% DC 5-7139 und 0.5% Silsoft Q führte zu einer erhöhten Pflegeperformance besonders bei der Nasskämmbarkeit und Weichheit im trockenen Haar im Vergleich zu 1.5% DC 5-7139 allein und ohne den Nachteil der statischen Aufladung. Aufgrund der extremstarken Aufladung der Spülungsvariante mit 1.5% Silsoft Q an den einzelnen Haarsträhnen wurde diese nicht im Halbseitentest evaluiert.

Besonders wichtig sind Silikone mit einem hohen Molekulargewicht, wie z.B. lineare DC 200 Fluid 60,000cst, 100,000cst sowie auch vorteilhaft hohe Partikelgrößen >15µm. Auch Mischungen von Amodimethikonen und dem kationischen Aminosilikon Terpolymer führen zu einer überragenden Pflegeperformance. Vorteilhaft ist der Einsatz des Silsoft Q Rohstoffs in der Konzentration 0.25 -1.0% Gewicht aktiv zusammen mit einem Überschuss an einem zweiten Silikon.

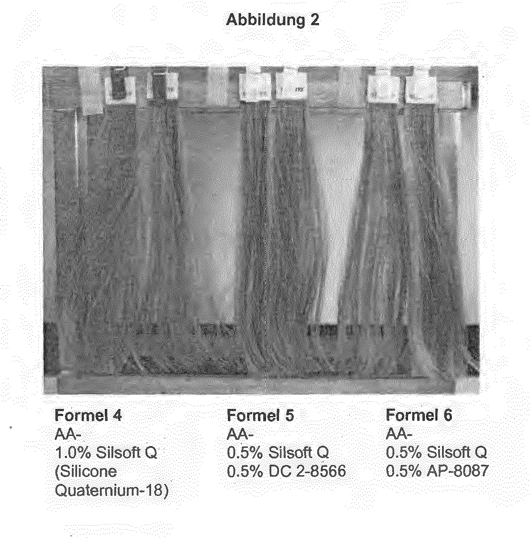

### Bild 2 (siehe Abbildung 2)

Vergleich Silsoft Q (2 Strähnen links, Formel 4) mit einem einer Mischung von 0.5% Silsoft Q / 0.5% DC 2-8566 (2 Strähnen mitte, Formel 5) und einer Mischung von 0.5% Silsoft Q / 0.5% AP-8087 nach Spülungsbehandlungen (2 Strähnen rechts, Formel 6). - 3x Kämmen Wie in Bild 2 abgebildet, Strähnen, die mit einer Spülung enthaltend 1.0% Silsoft Q behandelt wurden und danach 1x und 3x mit der feinen Seite eines Kamms (Typ 623.7 Hercules Sägemann ® 394.7) gekämmt wurden, zeigten eine viel stärkere Aufladung auf als die, die mit einer Spülung enthaltend eine Mischung aus 2 Aminosilikonen.

| | inci | **4** | **5** | **6** |
|---|---|---|---|---|
| 1 | Methylparaben | 0,25 | 0,25 | 0,25 |
| 2 | Propylparaben | 0,10 | 0,10 | 0,10 |
| 3 | Aqua | 83,44 | 83,70 | 83,70 |
| 4 | Cetyl Alcohol | 2,20 | 2,50 | 2,50 |
| 5 | Trisodium EDTA | | 1,00 | 1,00 |
| 6 | Lactic Acid | 0,80 | 0,84 | 0,84 |
| 7 | Sodium Chloride | 0,01 | 0,01 | 0,01 |
| 8 | Stearyl Alcohol | 4,40 | 4,80 | 4,80 |
| 9 | Oryzanol | 0,05 | 0,05 | 0,05 |
| 10 | Coco Betaine | 0,85 | 0,85 | 0,85 |
| 11 | Stearamidopropyl Dimethylamine | 2,20 | 2,20 | 2,20 |
| 12 | Water (and) Silicone Quaternium-18 (and) Trideceth-6 (and) Trideceth-12 | 5,00 | 2,50 | 2,50 |
| 13 | Parfum | 0,70 | 0,70 | 0,70 |
| 14 | Amodimethicone | | 0,50 | |
| 15 | Amodimethicone | | | 0,50 |

| **Tabelle 3** | **Handelsnamen der Rohstoffe:** |
|---|---|
| 1 | Nipagin M, Clariant, 99.5% |
| 2 | Nipasol M, Clariant, 99.5% |
| 4 | Nacol 16-95, Sasol , 95% |
| 5 | Trisodium EDTA Solution, 20%, Beiersdorf |
| 6 | 100366 Milchsäure 90, reinst DAB, Merck, 90% |
| 8 | Lanette 18, Cognis , 100% |
| 9 | Gamma -Oryzanol, Biesterfeld, 98% |
| 10 | Dehyton AB 30, Cognis, 31% |
| 11 | Tego Amid S18, Evonik Goldschmidt, 100% |
| 12 | Silsoft Q, Momentive Performance Materials, 20% |
| 14 | DC 2-8566, 100% Amodimethicone, Dow Corning |
| 15 | AP-8087, Dow Corning, 100% |

Formeln **4** und **5** (Tabelle 3) wurden im Halbseitentest im Haarstudio an trockenstrapazierten Haaren (N=10 Probanden) getestet und die Ergebnisse sind in Tabelle 4 abgebildet.

| **Haarstudio Daten; Halbseiten-Test an trocken/strapazierten Haaren, N=10 Probanden** | | |
|---|---|---|
| | **4** | **5** |
| Gehalt verteilen | 6,60 | 7,10 |
| Gleitvermögen verteilen | 7,40 | 7,50 |
| Gehalt sofort Ausspülen | 4,70 | 5,40 |
| Gleitvermögen sofort Ausspülen | 6,90 | 7,50 |
| Entwirrbarkeit nass | 6,70 | 7,50 |
| Kämmbarkeit nass | 7,10 | 7,60 |
| Haarstruktur nass Längen | 6,80 | 7,70 |
| Haarstruktur nass Spitzen | 5,90 | 6,90 |
| Gleitvermögen nass | 6,80 | 7,70 |
| Entwirrbarkeit trocken | 7,00 | 7,40 |
| Kämmbarkeit trocken | 7,70 | 7,80 |
| Gleitfähigkeit trocken | 7,50 | 7,80 |
| Struktur trocken Längen | 7,40 | 7,80 |
| weich, geschmeidig trocken | 2,00 | 5,00 |

Auch hier führt die Silikonmischung von 0.5% DC 2-8566 und 0.5% Silsoft Q zu einer erhöhten Pflegeperformance besonders im nassen Haar sowie auch bei der Weichheit im trockenen Haar im Vergleich zu 1.0% Silsoft Q allein und ohne den Nachteil der statischen Aufladung. Weiter erfindungsgemäße Formelbeispiele sind hiermit abgebildet:

| | inci | **7** | **8** | **9** | **10** | **11** | **12** | **13** |
|---|---|---|---|---|---|---|---|---|
| 1 | Methylparaben | 0,25 | 0,25 | 0,25 | 0,25 | 0,25 | 0,25 | 0,25 |
| 2 | Propylparaben | 0,10 | 0,10 | 0,10 | 0,10 | 0,10 | 0,10 | 0,10 |
| 3 | Aqua | Ad. 100 | Ad. 100 | Ad. 100 | Ad. 100 | Ad. 100 | Ad. 100 | Ad. 100 |
| 4 | Citric Acid | | 0,01 | 0,01 | | | | |
| 5 | Cetearyl Alcohol | | 3,50 | 4,44 | | | | |
| 6 | Cetyl Alcohol | 2,50 | | | 2,50 | 2,50 | 2,50 | 2,50 |
| 7 | Trisodium EDTA | | | | | | 1,00 | |
| 8 | Lactic Acid | 0,80 | | | 0,80 | 0,80 | 0,84 | 0,80 |
| 9 | Glycerin | | | 5,00 | | | | |
| 10 | Sodium Hydroxide | | 0,03 | 0,03 | | | | |
| 11 | Sodium Chloride | 0,01 | | | 0,01 | 0,01 | 0,01 | 0,01 |
| 12 | Stearyl Alcohol | 4,80 | | | 4,80 | 4,80 | 4,80 | 4,80 |
| 13 | Cetrimonium Chloride | | 2,00 | | | | | |
| 14 | Distearoylethyl Hydroxyethylmonium Methosulfate | | | 2,86 | | | | |
| 15 | Oryzanol | 0,05 | | 0,05 | 0,05 | 0,05 | 0,05 | 0,05 |
| 16 | Palmitamidopropyltrimonium Chloride | | | 1,66 | | | | |
| 17 | Coco Betaine | 0,85 | | | 0,85 | 0,85 | 0,85 | 0,85 |
| 18 | Stearamidopropyl Dimethylamine | 2,00 | | | 1,50 | 2,00 | 2,20 | 2,20 |
| 19 | Water (and) Silicone Quaternium-18 (and) Trideceth-6 (and) Trideceth-12 | 5,00 | 5,00 | 5,00 | 5,00 | 5,00 | 2,50 | 7,50 |
| 20 | Behentrimonium Methosulfate | | 2,73 | | | | | |
| 21 | Dimethicone | | | | 1,54 | 3,08 | | 2,31 |
| 22 | Bis-Hydroxy/Methoxy Amodimethicone | | | | | | 0,50 | |

| | **Handelsnamen der Rohstoffe:** |
|---|---|
| 1 | Nipagin M, Clariant, 99.5% |
| 2 | Nipasol M, Clariant, 99.5% |
| 4 | DSM Nutritional Products Europe, 100% |
| 5 | Nafol 1618-JA Sasol, 100% |
| 6 | Nacol 16-95, Sasol, 95% |
| 7 | Trisodium EDTA Solution, 20%, Beiersdorf |
| 8 | 100366 Milchsäure 90, reinst DAB, Merck, 90% |
| 9 | Glycerol EP, vegetable, Spiga Nord, 99.7% |
| 12 | Lanette 18, Cognis , 100% |
| 13 | CTAC 6025 KC, KCl, 25% |
| 14 | Dehyquart F 75, Cognis, 69.935% |
| 15 | Gamma -Oryzanol, Biesterfeld, 98% |
| 16 | Varisoft PATC, Evonik Goldschmidt, 60% |
| 17 | Dehyton AB 30, Cognis, 31% |
| 18 | Tego Amid S18, Evonik Goldschmidt, 100% |
| 19 | Silsoft Q, Momentive Performance Materials, 20% |
| 20 | Incroquat Behenyl TMS-50, Croda, 55% |
| 22 | AP-8087, Dow Corning, 100% |

## Patentansprüche

1. Zubereitung zur Behandlung keratinischer Fasern enthaltend
- ein kationisches Aminosilikon Terpolymer mit quaternären Ammonium-Gruppen und Polyethergruppen,
- ein Dimethikon und/oder ein zweites Aminosilikon,
- ein kationisches Tensid,
- eine oder mehrere Fettverbindungen mit Schmelzpunkt > 40°C,
- und einen wässrigen Träger
**dadurch gekennzeichnet, dass**
als kationisches Aminosilikon Terpolymer mit quaternären Ammonium-Gruppen und Polyethergruppen Silikon Quaternium-18 gewählt wird und
als Dimethikon ein hochmolekulares Dimethikon mit einer Viskosität von 60.000 bis 500.000 cSt gemessen mit einem Brookfield Viscosimeter RVF, RVT oder RVDV-II + gewählt wird.

2. Zubereitung nach Anspruch 1, **dadurch gekennzeichnet, dass** das zweite Aminosilikon eine Viskosität von 1000cst bis 6000cst, vorzugsweise von 1000cst bis 5000cst gemessen mit Brookfield Viscosimeter RVF, RVT oder RVDV-II + aufweist.

3. Zubereitung nach einem der vorangehenden Ansprüche, **dadurch gekennzeichnet, dass** als kationisches Tensid Stearamidopropyl Dimethylamine verwendet wird.

4. Zubereitung nach einem der vorangehenden Ansprüche, **dadurch gekennzeichnet, dass** das kationische Aminosilikon Terpolymer mit quaternären Ammonium-Gruppen und Polyethergruppen in Konzentrationen von 0.25 bis 1.5 Gew.-%, bevorzugt von 0.5 bis 1.0 Gew.-%, eingesetzt wird.

5. Zubereitung nach Anspruch 3, **dadurch gekennzeichnet, dass** das Dimethikon und/oder ein zweites Aminosilikon in gleichen oder höheren Konzentrationen, bevorzugt 1.5:1, 2:1, als das kationische Aminosilikon Terpolymer mit quaternären Ammonium-Gruppen und Polyethergruppen, jeweils bezogen auf den Aktivgehalt, eingesetzt wird.

6. Verwendung von Dimethikon und/oder ein zweitem Aminosilikon nach einem der vorangehenden Ansprüche zur Verringerung der statischen Aufladung von mit kationischem Aminosilikon Terpolymer mit quaternären Ammonium-Gruppen und Polyethergruppen nach einem der vorangehenden Ansprüche, kationischem Tensid nach einem der vorangehenden Ansprüche und wenigstens einer Fettverbindung mit Schmelzpunkt > 40°C nach einem der vorangehenden Ansprüche in einem wässrigen Träger behandelten keratinischen Fasern.

## Claims

1. Preparation for treating keratin fibres, containing
- a cationic aminosilicone terpolymer containing quaternary ammonium groups and polyether groups,
- a dimethicone and/or a second aminosilicone,
- a cationic surfactant,
- one or more fatty compounds having a melting point > 40°C,
- and an aqueous carrier,
**characterized in that**
the cationic aminosilicone terpolymer containing quaternary ammonium groups and polyether groups that is selected is silicone quaternium-18 and
the dimethicone that is selected is a high-molecular-weight dimethicone having a viscosity of from 60 000 to 500 000 cSt, measured using an RVF, RVT or RVDV-II+ Brookfield viscometer.

2. Preparation according to Claim 1, **characterized in that** the second aminosilicone has a viscosity of from 1000 cSt to 6000 cSt, preferably from 1000 cSt to 5000 cSt, measured using an RVF, RVT or RVDV-II+ Brookfield viscometer.

3. Preparation according to either of the preceding claims, **characterized in that** the cationic surfactant that is used is stearamidopropyl dimethylamine.

4. Preparation according to any of the preceding claims, **characterized in that** the cationic aminosilicone terpolymer containing quaternary ammonium groups and polyether groups is used in concentrations of from 0.25 to 1.5% by weight, preferably from 0.5 to 1.0% by weight.

5. Preparation according to Claim 3, **characterized in that** the dimethicone and/or a second aminosilicone is used in concentrations which are the same as or higher, preferably 1.5:1, 2:1, than the cationic aminosilicone terpolymer containing quaternary ammonium groups and polyether groups, based in each case on the active content.

6. Use of dimethicone and/or a second aminosilicone according to any of the preceding claims for reducing the static charge of keratin fibres treated with cationic aminosilicone terpolymer containing quaternary ammonium groups and polyether groups according to any of the preceding claims, cationic surfactant according to any of the preceding claims and at least one fatty compound having a melting point > 40°C according to any of the preceding claims in an aqueous carrier.

## Revendications

1. Préparation pour le traitement de fibres kératiniques, contenant
- un terpolymère cationique d'aminosilicone présentant des groupes d'ammonium quaternaire et des groupes polyéther,
- un diméthicone et/ou un deuxième aminosilicone,
- un tensioactif cationique,
- un ou plusieurs composés gras présentant un point de fusion > 40°C
- et un support aqueux
**caractérisée en ce qu'**on choisit, comme terpolymère cationique d'aminosilicone présentant des groupes d'ammonium quaternaire et des groupes polyéther, le Silicone Quaternium-18 et, comme diméthicone, un diméthicone de haut poids moléculaire, présentant une viscosité de 60.000 à 500.000 cSt, mesurée à l'aide d'un viscosimètre Brookfield RVF, RVT ou RVDV-II+.

2. Préparation selon la revendication 1, **caractérisée en ce que** le deuxième aminosilicone présente une viscosité de 1000 cSt à 6000 cSt, de préférence de 1000 cSt à 5000 cSt, mesurée à l'aide d'un viscosimètre Brookfield RVF, RVT ou RVDV-II+.

3. Préparation selon l'une quelconque des revendications précédentes, **caractérisée en ce qu'**on utilise, comme tensioactif cationique, la stéaramidopropyldiméthylamine.

4. Préparation selon l'une quelconque des revendications précédentes, **caractérisée en ce que** le terpolymère cationique d'aminosilicone présentant des groupes d'ammonium quaternaire et des groupes polyéther est utilisé en des concentrations de 0,25 à 1,5% en poids, de préférence de 0,5 à 1,0% en poids.

5. Préparation selon la revendication 3, **caractérisée en ce que** le diméthicone et/ou un deuxième aminosilicone est utilisé en des concentrations identiques ou supérieures, de préférence 1,5:1, 2:1, à celles du terpolymère cationique d'aminosilicone présentant des groupes d'ammonium quaternaire et des groupes polyéther, à chaque fois par rapport à la teneur active.

6. Utilisation de diméthicone et/ou d'un deuxième aminosilicone selon l'une quelconque des revendications précédentes pour la diminution de la charge statique de fibres kératiniques traitées par un terpolymère cationique d'aminosilicone présentant des groupes d'ammonium quaternaire et des groupes polyéther selon l'une quelconque des revendications précédentes, un tensioactif selon l'une quelconque des revendications précédentes et au moins un composé gras présentant un point de fusion > 40°C selon l'une quelconque des revendications précédentes dans un support aqueux.
